# EUROPEAN PATENT SPECIFICATION

(11) **EP 1 890 814 B1**
(45) Date of publication and mention of the grant of the patent: **08.05.2013**
(21) Application number: 06743266.6
(22) Date of filing: 07.04.2006
(51) Int. Cl.: B01L 3/00, C12M 1/34, C12Q 1/00, G01N 33/487

(54) **ANALYSIS DEVICE WITH REPLACEABLE TEST FIELD SUPPORT**
ANALYSEVORRICHTUNG MIT ERSETZBARER TESTFELDSTÜTZE
DISPOSITIF D'ANALYSE AVEC SUPPORT A CHAMPS DE TEST REMPLAÇABLE

(30) Priority: 14.04.2005 DE 102005017364
(43) Date of publication of application: 27.02.2008
(73) Proprietor: Roche Diagnostics GmbH, 68305 Mannheim (DE); F. Hoffmann-La Roche AG, 4070 Basel (CH)
(72) Inventor: MARQUANT, Michael, 68309 Mannheim (DE); LUNGU, Mihail-Onoriu, 67365 Schwegenheim (DE)
(74) Representative: Stößel, Matthias
(86) International application number: PCT/EP2006/061449
(87) International publication number: WO 2006/108811

(56) References cited:
- DE-A1- 19 546 535
- GB-A- 2 386 949
- US-A- 6 132 683
- US-A1- 2003 070 917
- US-A1- 2004 194 295
- US-B1- 6 187 164

## Description

The invention relates to an analysis device, in particular with a replaceable test field support, for the electrochemical determination of an analyte in a human bodily fluid, for example blood, several times in succession.

### Prior Art

US 5,286,362 relates to a method and a sensor electrode system for the electrochemical determination of an analyte or of oxidoreductase, as well as to the use of suitable substances for the electrochemical determination. Electrons are transferred in the presence of an oxidoreductase and a reducible substance, and these are transferred in the course of the determination reaction from the oxidoreductase to an electrode. This produces a signal which allows determination of the analyte to be determined, the reducible substance being enzymatically reduced and oxidized at the electrode. The substance which is produced at the electrode by oxidation differs from the reducible substance originally used. A corresponding sensor electrode system and components suitable for it are furthermore disclosed. According to US 5,286,362, a mixture of an oxidoreductase and a first reducible substance is used, the reducible substance having the property that this reducible substance is reduced by the oxidoreductase and produces a reduced substance in an irreversible reaction, and this reduced substance produces by oxidation a second reducible substance, which differs from the first reducible substance. An instrument is provided for holding the mixture of the oxidoreductase and the first reducible substance, as well as instruments for bringing the mixture in contact with a liquid sample, which may contain the analyte. Contact instruments are furthermore provided for electrical connection of the mixture upon contact with the sample to two electrical leads physically separate from each other. The contact instruments enclose an electrically conductive surface for receiving the electrodes from the first reducible substance, when the first reducible substance is reduced by the oxidoreductase and the reduced substance is produced, and for oxidizing the reduced substance at the electrically conductive surface to form a second reducible substance, which differs from the first reducible substance.

US 6,027,689 relates to a test card for optical or electrical determination of the concentration of a substance in a liquid. A test card is provided for use in a measuring device for evaluating the concentration of a substance in a liquid, for example a substance in a bodily fluid, which comprises a number of layers of material that is preferably suitable for being united in a continuous process during which the layers are unrolled from corresponding storage rolls. Each test card comprises a number of individually usable test sections, which are joined together and arranged in a successive sequence in the length direction of the test card. The material layers, of which the test card consists, enclose at least one reaction layer and a cover layer covering it, the cover layer comprising an opening for receiving a drop in each test section. A distribution layer and a carrier material layer are provided, which can likewise be incorporated in the test card, the carrier layer in each test section comprising a measurement opening which coincides with the opening for the drop. Weakened zones in the material, which are formed between neighbouring test sections, allow simple removal of an already used test section from the remaining test sections.

WO 2004/030822 A1 discloses a multiple capillary sensor analysis system. The capillary sensor analysis system is used to analyse a sample liquid with respect to an analyte contained in it, in particular for analysing a human or animal bodily fluid. A capillary sensor comprises a capillary channel, enclosed by at least two wall parts, having an inlet opening for the sample liquid and a vent opening. The capillary channel contains reagents, the reaction of the sample liquid with the reagents leading to a measurable change of a measurement quantity characteristic of the analysis. An evaluation device is provided, which comprises a capillary sensor frame for positioning a capillary sensor in a measuring position in order to carry out an analysis. The positioning is carried out so that the inlet opening of the capillary channel is accessible, in order to bring a sample liquid to be studied in contact with the inlet opening, the sample liquid entering the capillary channel because of capillary forces and filling it. Measurement and evaluation electronics are furthermore provided in order to measure the measurement quantity and determine the intended analysis information on the basis of the measurement value resulting from the measurement. The capillary sensors are designed as multiple capillary strips with a number of successively arranged capillary sensors. A multiple capillary sensor strip is guided and held in the capillary sensor frame of the evaluation device so that a capillary sensor of the strip respectively lies in the measuring position and its inlet opening is accessible for contact with sample liquid. The multiple capillary sensor strip can be moved in the evaluation device so that consecutive capillary sensors of the multiple capillary sensor strip can be transported into the measuring position. The evaluation device comprises a cutting instrument by which, after carrying out each measurement, the capillary sensor used for the measurement is cut from the multiple capillary sensor strip. Capillary sensors of the multiple capillary sensor strip are designed as electrochemical capillary sensors which respectively comprise a working electrode, a counter electrode and sensor contacts which are connected to the electrodes via conductor tracks and are in contact with corresponding device contacts of the evaluation device during the measurement, in order to establish an electrical connection to the measurement and evaluation electronics.

The above-discussed solutions according to the prior art represent arrangements of test field supports in which a plurality of individual test fields are arranged in a row on a band or a bar, and a new test field respectively has to be transported to the sample application position. In this case, either the respectively necessary electrical contact is established simultaneously or all the measuring cells are initially connected through by electrode pairs, and the respectively used individual test fields are removed. Transporting a sample application position to a fixed application position in the analysis set entails relatively high outlay, which is inherent to all the above-discussed solutions of the prior art. The user of the above-discussed arrangements is respectively offered only one test section, which is arranged on a band-shaped or strip-shaped support, and they are compelled to use the respectively available test field under the respectively available test field section. The user is restricted in terms of their selection opportunities, and can use only the respectively presented test section. Selection of test field sections, which could be employed in an arbitrary order by the user, is not possible with the above-discussed solutions known from the prior art.

### Description of the Invention

In view of the solutions known from the prior art, it is an object of the invention to provide an analysis unit which allows substantially simplified handling by an end-user, for example a diabetic, and is constructed much more simply than the solutions known from the prior art.

According to the invention, this object is achieved by the features of independent Claim 1.

The solution proposed according to the invention is advantageously distinguished in that a plurality of individual test fields or test sections are arranged in the form of a matrix on a test field support, so that the individual test fields can be used in any order. According to the alternative embodiments of the proposed solution, the position of the individual test fields on the test field support, and therefore the position of the individual test fields with respect to the measuring system, is fixed, it being possible to apply the sample at the available different positions of the individual test fields. This avoids the transport of a sample application position to a fixed application position, which can only be carried out with considerable outlay, which greatly simplifies the structure of the analysis unit or the measuring device.

In contrast to the use of individual test field supports, the handling outlay is significantly reduced with the solution proposed according to the invention by inserting a test field support on which a plurality of individual test fields are provided. A plurality of individual test fields are preferably arranged in matrix form on the side of the test field support accessible to the user. The number of individual test fields arranged on a single test field support, configured so that it can be replaced, is for example adapted to the number of glucose measurements required over a day by a diabetic. A number of individual test fields on a test surface, corresponding to this number, considerably reduces the outlay for carrying out a single measurement. At the same time, this solution avoids disposing of individual used test strips, possibly at different positions, which are otherwise separated from a continuous material according to the prior art.

In contrast to the individual test fields or bars in row form known from the prior art, arranged on bands to be transported through the analysis unit, the solution proposed according to the invention avoids complex mechanical or electromechanical drives which take up installation space, as well as the transport mechanisms necessary for them. The analysis units or analysis systems can therefore be designed to be much smaller, in particular significantly flatter. Avoiding mechanical or electromechanical drives and transport mechanisms, which take up installation space, furthermore makes it possible to produce such analysis units at much lower costs. In principle, the analysis units proposed according to the invention are less susceptible to perturbation and, compared with systems in which electrical drives are used, can be operated with smaller batteries or with such batteries as have a significantly extended life.

The analysis units proposed according to the invention, which receive the test field supports configured so that they can be replaced, advantageously stand out from the solutions known from the prior art in that the same parameter of the sample can be determined successively by all the individual test fields arranged on a replaceable test field support.

The analysis unit proposed according to the invention is distinguished by its simplified handling, an electrical contact between the analysis unit and the test field support, which can be inserted into it, being established when the test field support is inserted into a housing depression or a differently configured housing facility. The electrodes of all the electrochemical measuring cells of all the individual test fields of a test field support, configured so that it can be replaced, are always connected simultaneously to a common electrical measurement and control circuit. The analysis unit automatically detects which of the individual test fields matricially applied on the test field support is just to be used. Interconnection of the individual test field just to be used with the measurement and control circuit of the analysis unit takes place electronically in order to carry out the determination measurement.

After respective single use of all the individual test fields of a given test field support, i.e. after it has been fully used up, it is removed from the analysis unit.

In a first preferred alternative embodiment, the analysis unit with a test field support configured so that it can be replaced may be configured in the form of a fold-down case in the housing depression of which, formed on a lower shell, a credit-card-shaped, i.e. very flatly formed, test field support comprising a plurality of matricially arranged individual test fields can be inserted. After insertion, the very flatly formed test field support is electrically connected by an electrical contact strip formed on its rear side to an electrical contact strip designed complementarily with it in the housing depression in the lower side of the analysis unit designed in the form of a case.

According to this preferred alternative embodiment, a plurality of individual test fields are arranged in the form of a matrix on the surface of the test field support on the upper side which is accessible to the user after opening the upper shell.

The test field support comprises a stiffly designed support sheet, on which at least two electrode surfaces are respectively applied at the respective individual test field positions by methods such as laser ablation, lithography and screen printing. These are connected by conductor tracks to electrical contact surfaces at the edge of the test field support designed in the form of a card. A reagent layer is applied over the surface of the base sheet onto the electrode and conductor track structures. This reagent layer contains the reagents necessary for specific detection of the intended parameters from the sample. There is a spacer sheet on the reagent layer, which may for example be adhesively bonded onto the reagent layer. The spacer sheet comprises holes at the positions of the electrodes, which form measuring chambers. A cover sheet is furthermore applied on the spacer sheet. The cover sheet may, for example, be adhesively bonded onto the spacer sheet. The cover sheet seals the measuring chambers and is designed so as to create openings for receiving the sample at the measuring positions, as well as respectively a vent hole. Above the cover sheet, there is a sealing sheet for closure, by which the measuring capillary spaces that are formed are externally sealed in a moisture-tight fashion.

In order to carry out the measurement, one of the measuring chamber dosing openings is respectively opened by the user, which may for example be done by removing a ring-pull closure or a tab or the like, and they then apply the sample for the measurement.

Before the first individual test field on the test field support designed in the form of a card can be used for the measurement, the test field support designed in the form of a card is inserted into a measuring device, for example designed in the form of a case, in order to carry out the first measurement, to which end it has an indentation designed in the form of a depression. In order to insert the test field support designed in the form of a card, and to carry out a measurement, it is possible to fold open the upper shell of the analysis unit designed in the form of a case, which has a display on its inside that the user can read when it is in the folded open state.

The test field support designed in the form of a card is inserted with its lower side comprising an electrical contact strip into the recess designed in the form of a depression in the lower shell of the analysis unit, which may be designed in the form of a case. After the measurement, the analysis unit is deactivated by folding the upper shell shut. Activation of the analysis unit may be carried out in a straightforward way by folding the upper shell open.

The electrodes of all the individual test fields arranged on the upper side of the test field support designed in the form of a card are simultaneously connected to the contacts of the analysis unit. A measuring cell may be selectively connected up to the measurement and control circuit of the device electronically using an analogue semiconductor switching matrix. After the analysis unit is activated, all the measuring electrodes of the individual test fields are serially electronically tested repeatedly by a conductance measurement. The analysis unit thereby detects which of the individual test fields has been provided with a sample, and then carries out the electrochemical detection measurement on the corresponding electrodes. The result is subsequently shown on the display arranged on the inside of the upper shell.

Another preferred alternative embodiment of the solution proposed according to the invention relates to a test field support comprising a continuous band, in which capillary sensors are formed. According to this alternative embodiment, however, the individual capillary sensors are not cut into individual test strips but are present as blocks in a mutually adjacent arrangement to form a plurality of capillary sensors. The band, preferably designed in a plurality of layers, is constructed from individual layer materials held on a plurality of rolls. On a strip base sheet, there are a conductive structure, the electrode surface, the conductor tracks and contacts. A strip-shaped reagent film with the reagents necessary for the intended detection reaction is applied onto the stiffer base sheet in the region of the electrode surfaces. On this, there is in turn a stamped spacer sheet which, on one side of the band, forms a capillary open to this side and a measuring cell above the electrode surface and, on the other side of the band, simultaneously exposes contact surfaces for electrical connection at the ends of the conductor tracks. A cover sheet, which seals the capillaries on the upper side and forms a vent hole at the inner end of the capillary, is adhesively bonded onto the spacer sheet for closure. If a moisture-isolating seal is optionally necessary in order to protect the reagent layer, then the band may be formed longer in front of the capillary side, in which case U-slot-shaped stamping around the front of the capillary leaves it open on the dosing side, but simultaneously forms a closed frame around the dosing opening. By sealing around the stamped region with a vapour-tight sheet, the capillary region with the reagents provided there is therefore protected against ingress of moisture.

In order to expose the capillary opening, the frame is bent down on the front edge, so that a protective sheet can be removed from the dosing opening.

Two band-shaped sections of the band constructed in layers, respectively with for example five individual test fields, i.e. five capillary measuring cells, are inserted opposite each other into an analysis unit. The analysis unit comprises a lower part, on which a block-shaped test field support is respectively placed on two opposite sides. Connected to the lower part of the analysis unit via a hinge, there is an upper part which is folded onto the lower part after inserting the test field supports, and therefore fixes the test field supports inserted into the analysis unit. At the same time, contact of the measuring cell electrodes with electronics in the upper part is established by resilient contacts correspondingly arranged in a row.

The display lies on the upper side of the folded-shut device; control buttons may furthermore be provided on the upper side. The analysis unit according to this second alternative embodiment is designed so that the openings for the sample application positions, i.e. the capillary openings of the block-shaped test field supports inserted into the analysis unit, face out on both sides and are therefore readily accessible.

The analysis unit may furthermore comprise an outer cover sleeve, which is applied by sliding over the analysis unit. In the slid-on state, this cover sleeve forms a handle for holding the analysis unit. Also according to this alternative embodiment, the electrodes of all the individual test fields of the test field support designed in the form of a block and containing a plurality of capillary measuring cells are simultaneously connected to the contacts of the analysis unit. Here as well, an individual measuring cell may be selectively connected up to the measurement and control circuit of the device electronically, for example using an analogue semiconductor switching matrix. After the analysis unit is activated, the measuring electrodes of the individual test fields are serially electronically tested repeatedly by a conductance measurement, so that the analysis unit or a measuring algorithm implemented in it detects which of the individual test fields has been provided with a sample. The electrochemical detection measurement is then carried out, and the result is subsequently shown on the display of the analysis unit After having used all the individual test fields of the test field support, which may be designed in the form of a block or in the form of a card, it may be removed from the analysis unit simply by folding open the upper part.

According to another alternative embodiment of the concept on which the invention is based, a test field support may be slid into a slot-in module, which can be slid into a sleeve designed complementarily with the configuration of the slot-in module and is securely accommodated in it. The slot-in module encloses a for example rectangular recess, into which the card-shaped test field support containing a plurality of individual test fields is inserted. The individual test fields or test sections, designed in the form of tongues, have capillary openings on an outer edge.

The individual test fields or test sections are in a mutually adjacent arrangement, and a light-emitting diode may be assigned to each individual test field. After having used all the individual test fields of a test field support designed in the form of a card, the used test field support may be removed by unlatching on the slot-in module and replaced by a new flatly designed test field support comprising unused individual test fields or test sections.

### Drawing

The invention will be described in more detail below with reference to the drawing, in which:
Figure 1 shows a representation of the first alternative embodiment of an analysis unit proposed according to the invention, with the card-shaped test field support inserted,
Figure 2 shows a cross section through the card-shaped test field support inserted into the analysis unit, with a representation of the electrode structure,
Figure 3 shows an analogue semiconductor switching matrix for electrical contact of the individual fields of the card-shaped test field support according to Figure 1,
Figure 4 shows a reproduction, represented on an enlarged scale, of the upper side of the test support with matricially arranged individual test fields, the first individual test field row of which is open,
Figure 5 shows a card-shaped test field support represented having been removed from a depression in the lower shell of the analysis unit,
Figure 6 shows the representation of a further alternative embodiment of the analysis unit proposed according to the invention, in the folded-open state,
Figure 7 shows the analysis unit according to Figure 6 before sliding into a cover sleeve,
Figure 8 shows an analysis unit according to the representation in Figure 6, slid into the cover sleeve,
Figure 9 shows a perspective view of an analysis unit configured as a slot-in module,
Figure 10 shows the slot-in module of the analysis unit according Figure 8 in the state extracted from the slot-in box and with the card-shaped test field support inserted,
Figure 11 shows an enlarged representation of the slot-in module with the test field support designed in the form of a card inserted,
Figure 12 shows the slot-in module, without the card-shaped test field support, removed from the slot-in box,
Figure 13 shows the layer structure of an individual test strip of the card-shaped test field support in an exploded representation, and
Figures 14.1 to 14.3 show a perspective representation of the test field support designed in the form of a card with individual test strips, with and without a protective sheet in the region of the terminal electrodes and in the region of the capillary openings.

### Alternative Embodiments

In what follows, the terms measuring devices or analysis units will be intended to mean portable devices which a user can always carry with them on their person. Such transportable measuring devices or analysis devices contain a long-term energy store, which supplies energy to evaluation electronics accommodated in the portable measuring devices or in the portable analysis unit. The test field supports which can be inserted into the portable measuring devices or analysis units are preferably a medical consumable material, which is removed from the device after use and replaced by a new test field support with unused individual test fields. As an alternative or in addition, however, it is also possible to employ multiply usable test field supports which, for example, can be regenerated again after each use so that they can subsequently be reused.

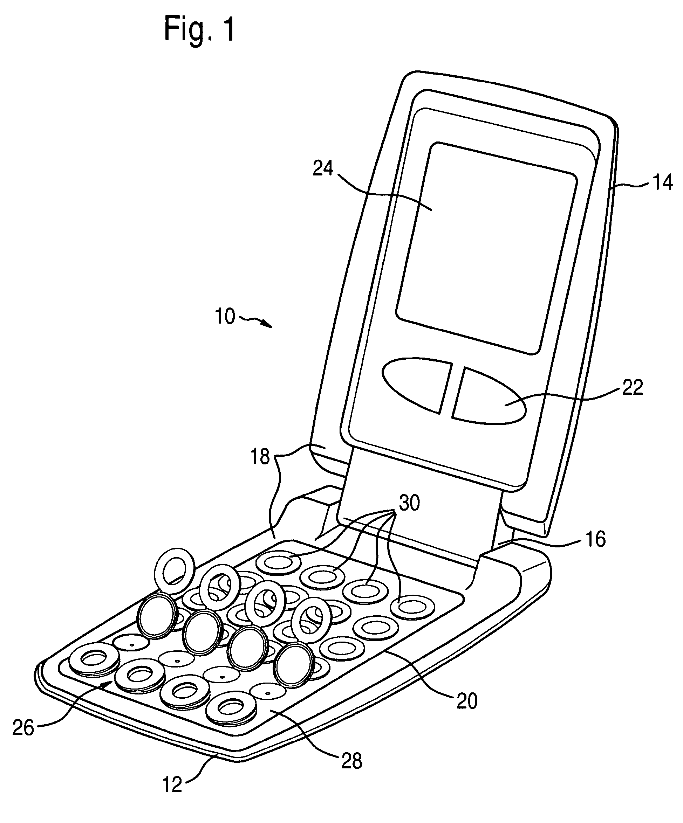

Figure 1 shows an analysis unit according to the present invention, having a card-shaped test field support inserted in an analysis unit.

The representation according to Figure 1 represents a measuring device or an analysis unit which comprises a lower shell 12 and an upper shell 14 connected by means of an articulated connection 16. An indentation in the form of a depression is formed in the lower shell 12, in which according to the representation in Figure 1 the flat card-shaped test field support 26 can be inserted. The boundary of the indentation in the form of a depression in the lower shell 12 is identified by the reference numeral 20. There are a first display 22 and a second display 24 in the upper shell 14 of the measuring device 10 or the analysis unit 10.

As can be furthermore seen from the representation according to Figure 1, the flatly designed card-shaped test field support 26 comprises a test support surface 28, on which a matricially configured test field array 30 is arranged. After the upper shell 14 of the measuring device 10 or the analysis device 10 is opened, all the individual test fields of the test field array 30 are accessible. Each individual test field of the test field array 30 is closed, and can be opened by the user as will be described in more detail below.

After the upper shell 14 is opened, the user can freely select which of the individual test fields of the matricially configured test field array 30 they wish to use, and use it after opening the closure. For diabetics, for example, this represents a very simple and user-friendly procedure since the number of blood sugar measurements required daily for diabetics can be carried out very straightforwardly with the individual test fields available in the test field array 30. Since, after a corresponding individual test field from the test field array 30 has been used, it remains inside the flatly designed test field support 26, an environmental burden due to discarded test strips, which may for example occur in the solutions according to the prior art, can be precluded.

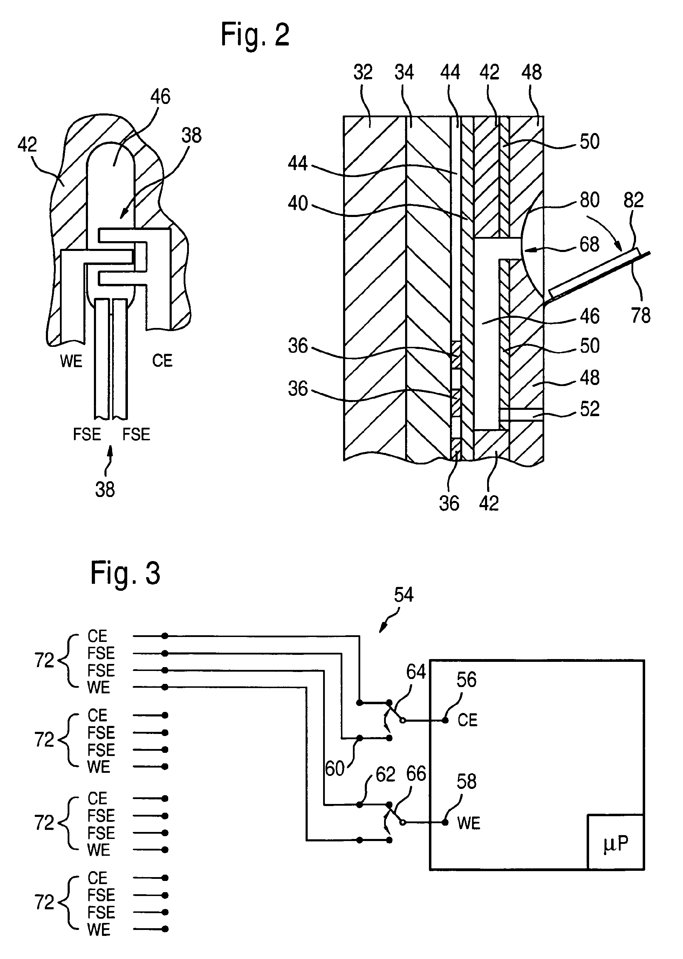

Figure 2 shows a cross section through the card-shaped test field support according to the representation in Figure 1, and a representation of the electrode structure.

It can be seen from the sectional representation according to Figure 2 that the test field support 26 designed flatly, preferably in the form of a card, has a layered structure.

A support sheet 34 is applied onto a base sheet 32. The support sheet 34 is covered by a reagent layer 40, a column-shaped hole 44 being formed between the side of the support sheet 34 facing the reagent layer 40. Conductor tracks 36 for the connection of electrodes 38 extend inside the column-shaped hole 44. The conductor tracks 36 are represented in section in the sectional representation according to Figure 2. The aforementioned reagent layer 40 lies above the conductor tracks 36. Between the reagent layer 40 and a spacer sheet 42, which covers the reagent layer 40 in individual regions, measuring chambers or measuring capillary spaces 46 are formed. The electrochemical measuring cells 46 are covered by a hydrophilic layer 50, which is in turn covered by a cover sheet 48. In order to vent the electrochemical measuring cells 46, both the cover sheet 48 and the hydrophilic layer 50 arranged below it are pierced by a vent 52.

The electrochemical measuring cell represented in section in Figure 2, or the electrochemical measuring cell 46 represented in Figure 2, are in communication via an opening with the lower side of an individual test field 68 having a reception well 80. In the representation according to Figure 2, a cover 78 closing the individual test field 68 is represented in its open position. On the side facing the reception well 80, the cover 78 has a sealing edge 82.

It can furthermore be seen from the representation according to Figure 2 that the electrochemical measuring cell 46, bounded by the spacer sheet 42 on the one hand and by the reagent layer 40 and the hydrophobic layer 50 on the other hand, accommodate for example electrodes 38 arranged mutually opposite. The electrodes 38 comprise a counter electrode CE and a further electrode WE. In the representation according to Figure 2, the ends of the electrodes CE and WE protrude into each other in the form of a comb. Electrodes FSE detecting the filling level of the electrochemical measuring cell 46 are furthermore provided, an individual electrochemical measuring cell 46 always respectively being assigned a pair of electrodes FSE detecting its filling level. In the representation according to Figure 2, the filling level electrodes FSE detecting the level to which the electrochemical measuring cell 46 is filled with a sample liquid protrude into the electrochemical measuring cell 46 in the region of the electrodes CE and WE.

The representation according to Figure 3 shows an analogue semiconductor switching matrix 54 in a schematic representation. The electrodes 38, CE and WE represented in Figure 2, as well as the electrodes FSE detecting the filling level of the electrochemical measuring cell 46 are integrated into an analogue semiconductor switching matrix 54. Each electrochemical measuring cell 46, and therefore each individual test field 68 of the test field support 26 designed flatly, preferably in the form of a card according to the sectional representation in Figure 2, are assigned four electrodes. To this end, an evaluation component comprises a CE terminal 56, at which the voltage can be applied to the CE electrode, and a WE terminal 58 to which the voltage can be applied on the WE terminal. Each of the terminals 56, 58 is assigned a switch 64 or 66. The first switch 64 switches between the CE electrode and a terminal 60 of the first filling level electrode, whereas the second switch 66 switches to and fro between the WE electrode and the terminal of the second filling level electrode 62. This arrangement ensures that the individual test field 68, respectively wetted by a sample and deliberately selected by the user, is evaluated by the evaluation component to which voltages conveyed by the electrodes are transmitted. The result recorded by the evaluation component is correspondingly shown graphically on the first display 22 represented in Figure 1 or the second display 24. The electrodes CE, WE represented in Figure 3 and the two FSE electrodes, via which the filling level of a bodily fluid such as whole blood or thinned blood in the electrochemical measuring cell 46 can be detected, are assigned to each of the individual test fields 68 arranged matricially in the card-shaped test field support 26. The two filling level electrodes FSE detecting the filling level in the electrochemical measuring cell 46 ensure that a measurement is always carried out with a sufficient liquid content in the electrochemical measuring cell 46, and that both the CE electrode and the WE electrode are fully wetted by the liquid containing the analyte.

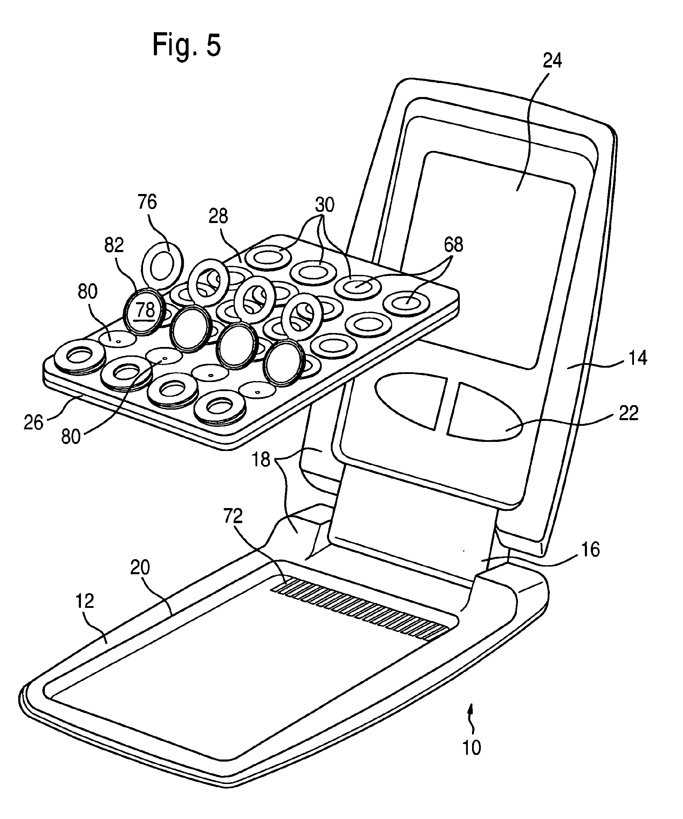

The electrodes CE, WE represented in Figure 3 and the two FSE electrodes are all connected to the electrical contact strip 72 which can be seen on the shell side in Figure 5. When the test field support 26 designed in the form of a card is inserted into the lower shell 12 of the analysis unit 10, the electrical contact strip 72 arranged in the lower shell 12 is connected with a complementarily configured electrical contact strip 70 on the lower side of the test field support 26 designed in the form of a card. In this way, the individual test fields 68 arranged matricially as a test field array 30 formed on the upper side of the flat test field support 26, designed in the form of a card, are electrically connected when the test field support 26 designed in the form of a card is inserted into the lower shell 12 of the measuring or analysis device 10. A microprocessor assigned to the analogue semiconductor switching matrix 54 switches through the analogue semiconductor switching matrix 54 so that it is possible to check to which of the individual test fields 68 an electrically conductive connection has been made, based on filling of the electrochemical measuring cell respectively assigned to this individual test field 68. Whether the filling level of a raw liquid in the relevant electrochemical measuring cell 46 is sufficient is then determined via the electrodes FSE, in order to ensure a reliable measurement result.

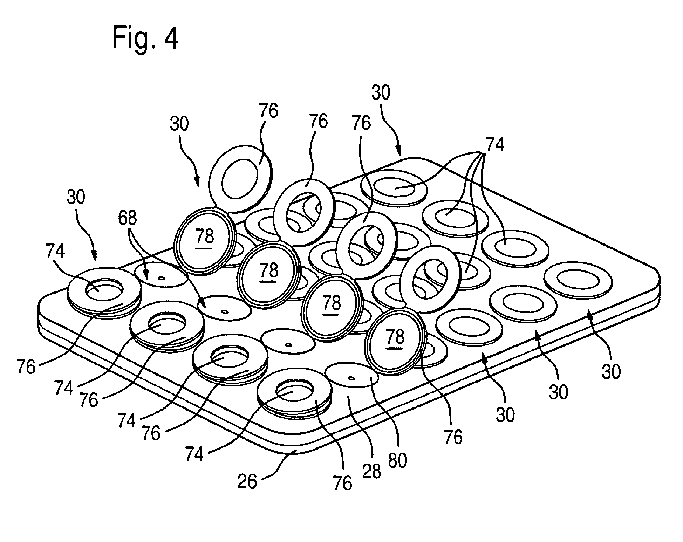

The representation according to Figure 4 shows a representation of the upper side of the flat test field support designed in the form of a card, on an enlarged scale.

In the representation according to Figure 4, the flat test field support 26 is removed from the depression-shaped indentation in the lower shell 12 of the measuring device 10 or the analysis unit 10, and represented separately. In the representation according to Figure 2, the great majority of the individual test fields 68 in the test field array 30 are closed, whereas one row of the individual test fields 68 formed matricially on the upper side of the flat test field support 26 is represented in the open state. In the closed state, each individual test field 68 is closed by a cover 78, each of the covers 78 having a sealing edge 82. In the closed state of the cover 78, the sealing edge 82 seals a reception well 80 on the flat test field support 26. In order to open the covers 78, they are respectively provided with a ring-pull closure 76, the tab of which protrudes slightly beyond the cover 78 in the state closing the reception well 80. In order to open an individual test field 68 and introduce a sample of bodily fluid, for example whole blood or thinned blood, the user pulls on a recloseable ring-pull closure 76 and brings the cover 78 into an upright position as represented in Figure 4. The ring-pull closure 76 can then be reclosed, so that the individual test field 68 is sealed.

Figure 5 shows a flat test field support which is removed from the indentation provided in the lower shell of the measuring device or the analysis unit in the folded-open state.

Figure 5 shows the depression-shaped indentation, which is formed in the lower shell 12 of the measuring device 10 designed in the form of a case. The replaceable flat test field support 26, removed according to the representation in Figure 5 from the lower shell 12, comprises individual test fields 68 which are accessible because the cover 78 is placed open, whereas the majority of the individual test fields 68 formed on the upper side of the flat test field support 26 are sealed by closed covers 78. The electrical contact with the flat test field support 26, configured so that it can be replaced, is carried out on the one hand via a contact strip 72 formed in the depression-shaped indentation of the lower shell 12, which interacts with an electrical contact strip formed complementarily with it on the lower side of the flat test field support 26. When the flat test field support 26 in the unused state is inserted into the depression-shaped indentation in the lower shell 12, contact with the flat test field support 26 therefore takes place directly.

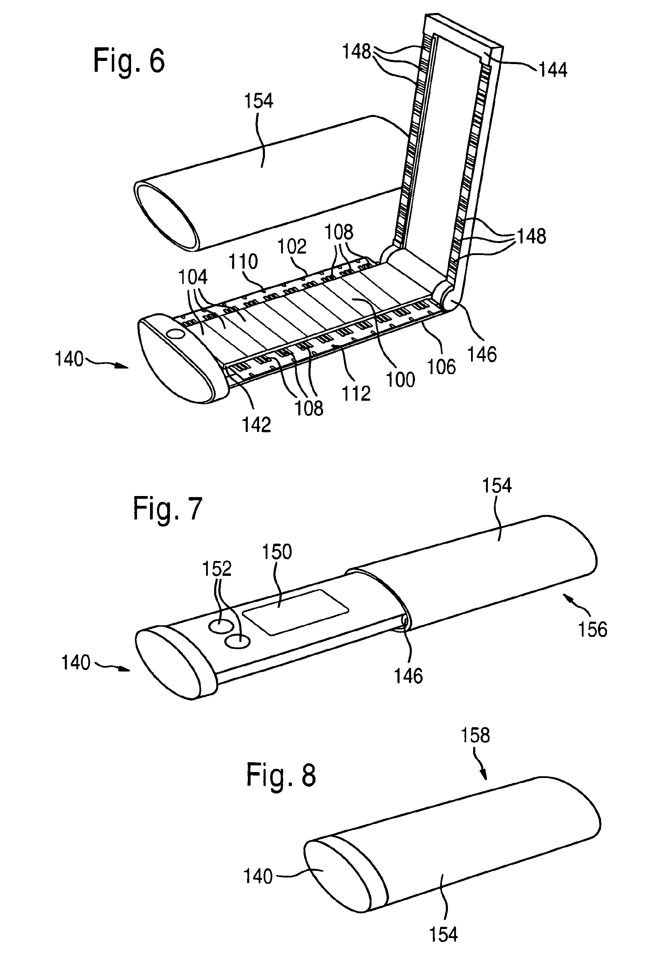

Figure 6 shows a second alternative embodiment of the concept on which the invention is based, the arrangement represented in Figure 6 likewise comprising two parts connected together at a hinge 146, i.e. a lower part 142 and an upper part 144. Figure 6 represents a corresponding measuring device or analysis unit 140 in the open state. A capillary sensor support 100 is inserted into the lower part 142, this having five or more capillary sensors 104 lying next to one another.

The band assembled from rolls comprises a plurality of layers, which create an electrochemical capillary sensor 104 when laminated together. The capillary sensor support 100 containing a plurality of layers in its final assembled state comprises a stiffer base sheet having a conductive structure, electrode surfaces, conductor tracks and contacts. A strip reagent film with the reagents necessary for the intended measurement reaction is applied over the more stiffly designed base sheet by flow coating in the region of the electrodes. A further layer is in turn applied on top in the form of a stamped spacer sheet, for example adhesively bonded. On one side of the capillary sensor support 100, it forms a capillary 108 open on this side as well as an electrochemical measuring cell over the electrode surfaces and, at the same time, on the other side of the band, conductor track contact surfaces at the ends, on which electrical contact can take place. A cover sheet, which seals the capillary 108 at the top and forms a vent hole at the inner end of the capillary 108, is finally adhesively bonded onto the applied, for example adhesively bonded reagent sheet.

Optionally, sealing of the reagent layer against moisture may be achieved in that the band is designed to be wider on the capillary side, in which case U-slot-shaped stamping around the front of the capillary 108 leaves it open on the dosing side, but simultaneously forms a closed frame around the dosing opening. By sealing around the stamped region with a thin vapour-tight sheet, the capillary region with the reagents can be protected against ingress of moisture.

In order to expose the capillary 108, the frame (not shown in Figure 6) is bent up on the front edge of the capillary sensor support 100, after which a protective sheet can be removed from the opening of the capillary 108. Two of these sections, for example each having five individual test fields (capillary measuring cells), may be inserted opposite each other into the analysis unit 140. The lower part 142 and the upper part 144 are closed, so that the inserted capillary sensor support 100 is immobilized and contact takes place with strip-shaped contact regions 110, 112 of the capillary sensor support 100, so that the measuring cell electrodes is established with electronics, which are accommodated in the upper part 144 of the measuring device 140 or the analysis unit 140, by resilient contacts 148 arranged in a row. The two contact regions 110 and 112, extending mutually parallel, run parallel to the first long side 102 or the second long side 106 of the capillary sensor support 100. The electronics test all the individual test fields of the capillary sensor support 100 as regards which of the capillary sensors 104 has been selected for use by the user, since a conductive connection is set up in the assigned electrochemical measuring cell 46 in the capillary sensor 104 being used, owing to the sample liquid which enters.

If the upper part 144 of the analysis unit 140, which connects the contact regions 110 and 112 of the capillary sensor support 100, is folded down about the hinge 146, then the contact regions 110, 112 are connected by resilient contacts 148 which are designed complementarily with the profile of the contact regions 110, 112 of the capillary sensor support 100 in the upper part 144. When the upper part 144 is folded down, i.e. an electrical connection is established between the resilient contacts 148 and the contact regions 110, 112 of the capillary sensor support 100, then the openings of the capillaries 108 protrude laterally beyond the folded-down upper part 144. The user of the connected capillary sensor support 100 can therefore select which of the capillary sensors 104 they use and - in contrast to the solutions known from the prior art - is not restricted to successive presentation of them.

Figure 7 shows the measuring device 140 folded together, or the analysis unit 140 folded together, before sliding into a cover sleeve.

In the representation according to Figure 7, the lower part 142 and the upper part 144 of the measuring device 140 or the analysis unit 140 connect with the capillary sensor support 100. On the upper side of the upper part 144, there are a display 150 and optionally control buttons, which are identified by the reference numerals 152. In the folded-together state, the measuring device 140 or the analysis unit 140 can be slid into the cover sleeve 154 (cf. Figure 8). In the slid-on state 156, the cover sleeve 154 is also used as a handle for holding the measuring device 140 or the analysis unit 140.

The electrodes of all the individual test fields or capillary sensors 104 of the capillary sensor support 100 are simultaneously connected to the resilient contacts 148 arranged in a row in the upper part 144. Deliberate determination of the respectively used electrochemical measuring cell for the measurement and control circuit of the measuring device or the analysis device 140 is carried out electronically via an analogue semiconductor switching matrix 54 (cf. Figure 3). After activation of the measuring device 140 or the analysis unit 140, all the measuring electrodes of the individual test fields are serially electronically tested repeatedly by a conductance measurement, so that the measuring device 140 detects, for example with the aid of a measurement algorithm implemented in it, which of the individual test fields has been dosed with a sample, for example in the form of a drop of whole blood, and then carries out the electrochemical detection measurement on the electrodes. Its result is subsequently shown on the display 150 on the upper side of the upper part 144.

Once all the individual test fields or capillary sensors 104 of the capillary sensor support 100 have been used, it is removed from the measuring device 140 or the analysis unit 140. To this end - as represented in Figure 7 - the cover sleeve 154 is slid fully off the measuring device 140, then a latch between the upper part 144 and the lower part 142 is released. After opening the upper part 144 and the lower part 142, the capillary sensor supports 100 can be taken out and replaced by unused, new usable capillary sensor supports 100.

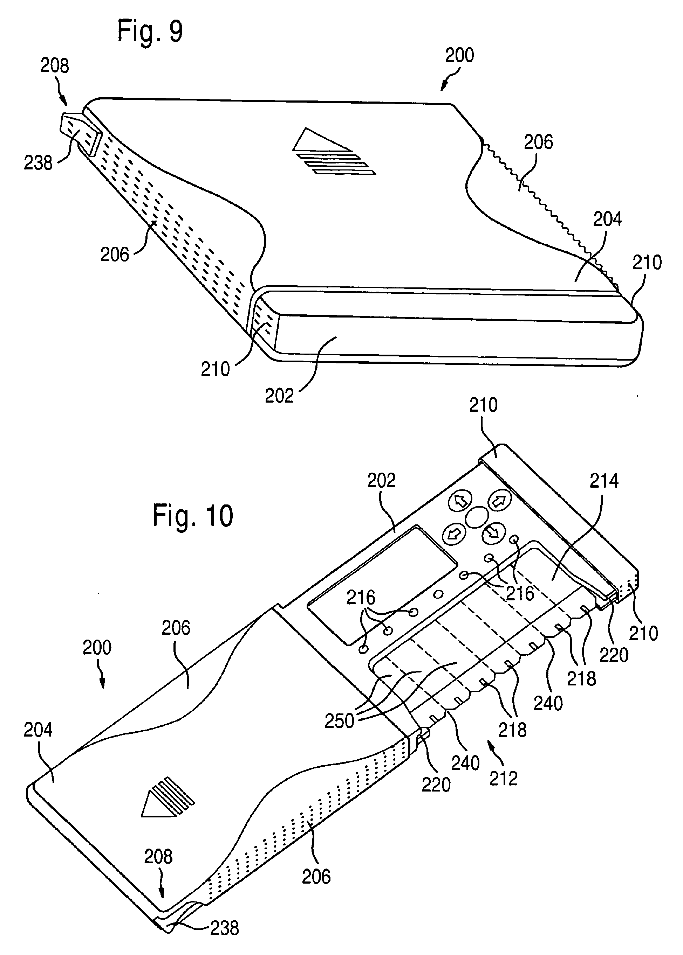

Figure 9 shows another alternative variant of the solution proposed according to the invention, with a slot-in module which can be slid into a slot-in box.

A slot-in arrangement 200 comprises a slot-in module 202 and a slot-in box 204. Gripping pieces 206 are externally applied on the slot-in box 204, and a latching/unlatching device 208 with a latching element 238 is furthermore located at one end of the slot-in box 204. Gripping surfaces 210 are formed on the extraction side of the slot-in module 202, using which the slot-in module 202 can be extracted from the slot-in box 204 after actuation of the latching/unlatching device 208.

Figure 10 shows the slot-in arrangement 200 with the slot-in module 202 extracted from the slot-in box 204. A recess 212, into which a flatly designed test field support 214 is slid, is formed on the slot-in module 202. The test field support 214 contains a plurality of test sections 250 which can respectively be separated from one another by a free space 240, designed in the form of a prong, and respectively have a capillary opening 218 for receiving a sample liquid. There are light-emitting diodes 216 on the upper side of the slot-in module 202, each of which is assigned to a test section 250 of the flatly designed test field support 214. The flat test field support 214 can be slid into the slot-in module 202 in slot-in slots 220, which are formed on the side surfaces of the recess 212. After the slot-in module 202 is extracted from the slot-in box 204, the individual capillary openings 218 are freely accessible. The user of the slot-in arrangement 200 can therefore select which of the test sections 250 of the test field support 214 they wish to use, and is not restricted to successive presentation of the individual test sections 250.

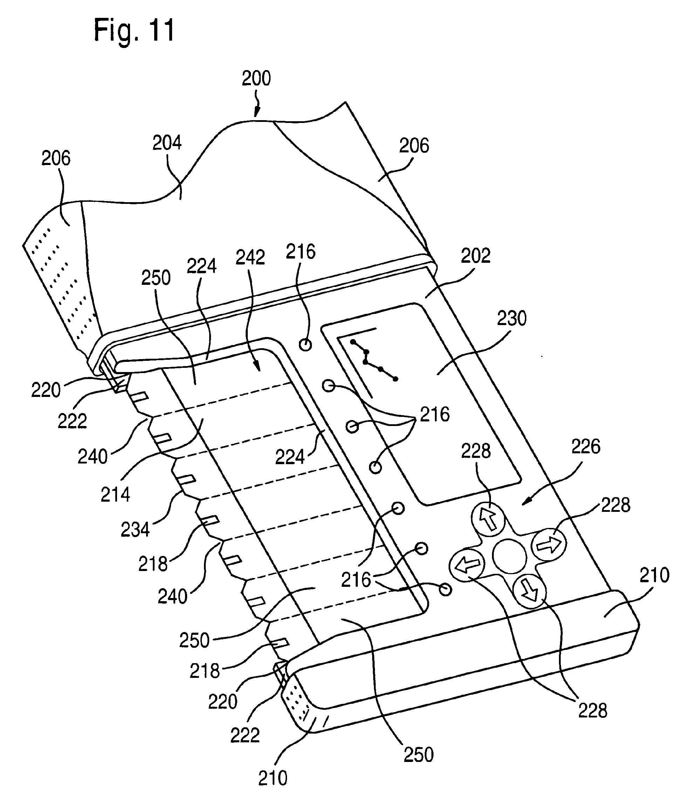

The representation according to Figure 11 shows the slot-in module on an enlarged scale.

The slot-in module 202 has a control field 226 and a plurality of buttons 228, which the user actuates, on its upper side in the vicinity of the light-emitting diodes 216. It can furthermore be seen from the representation according to Figure 11 that the flatly designed test field support 214 is slid laterally into the slot-in module 202 in the recess 212 bounded by a bounding wall 224, its outer edge 234 being freely accessible. Connection of the test field support 214 in the slot-in module 202 is carried out by fully sliding it in with its opposite side from the outer edge 234 into slot-in slots 220. The representation according to Figure 11 shows the protruding edges of a guide surface 222 below the outer edge 234 of the test field support 214. This facilitates the lateral sliding of the test field support 214 into slot-in slots 220 extending perpendicularly to the bounding wall 224. Each of the test sections 250 of the test field support 214 has a capillary opening 218, and the test sections 250 of the test field support 214 may be separated from one another by for example triangularly designed free spaces 240. When the flatly designed test field support 214 is slid laterally into the recess 212 and electrically connected to the slot-in module 202 in the slot-in module 202 at the end of the slot-in slots 220, the user can expose and use the capillary openings 218 arranged on the outer edge 234. The capillary openings 218 can be freely exposed before sliding into the slot-in module 202 by removing a sheet which seals and closes the capillary openings 218 before insertion. Sealing elements may furthermore be provided on the slot-in module 202, with which it is possible to seal the capillary openings 218.

The user is free in their choice of which of the test sections 250 of the test field support 214 is selected. Depending on the test field support 214 selected, which is produced application-specifically in respect of its reagents, and depending on the analyte with respect to which the sample liquid is to be studied, a wide variety of values such as cholesterol values, lactate values as well as blood sugar values and the like, can be shown in the display 230 by means of the control field 226 and the keypad 280 arranged there, so that these can be read off directly by the user.

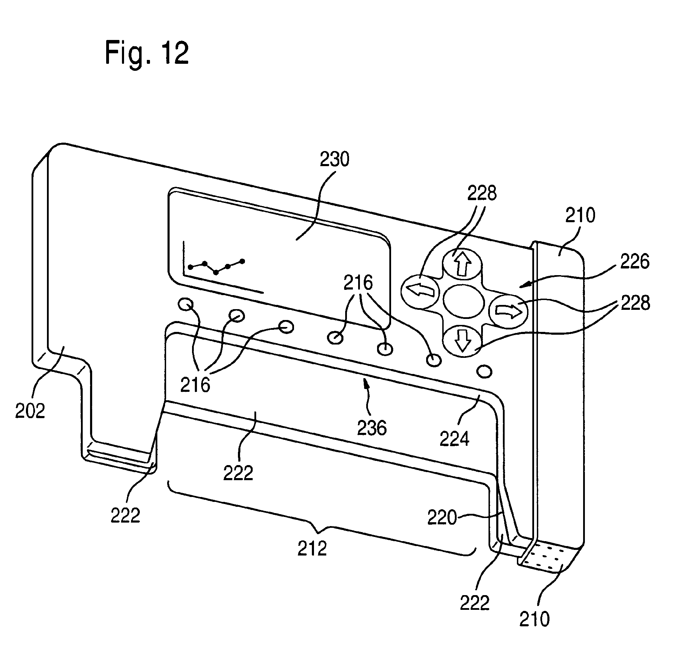

Figure 12 in turn shows a representation of the slot-in module 202 without a test field support 214 slid into the recess 212.

Between the slot-in slots 220, there is a guide surface 222, which extends inside the recess 212. Below the bounding wall 224, the test field support 214 (not shown in Figure 12) or the test sections 250 formed on it can be electrically connected in the slot-in module 202. The electrical contact region lies below the bounding wall 224 in the slot-in module 202 and is indicated by the reference numeral 236. A display 230 is arranged on the upper side of the slot-in module 202. Various values can be shown on the display 230 after actuating the keypad 228 arranged next to it, for example values for the cholesterol content, lactate values and values for the blood sugar content and the like.

The displays 24, 150 and 230 of the alternative embodiments described above may furthermore show which of the individual test fields 38 of the respective test field support 26, 100 and 214 has been used and/or which of the individual test fields 68, 104 and 250 is still available for use.

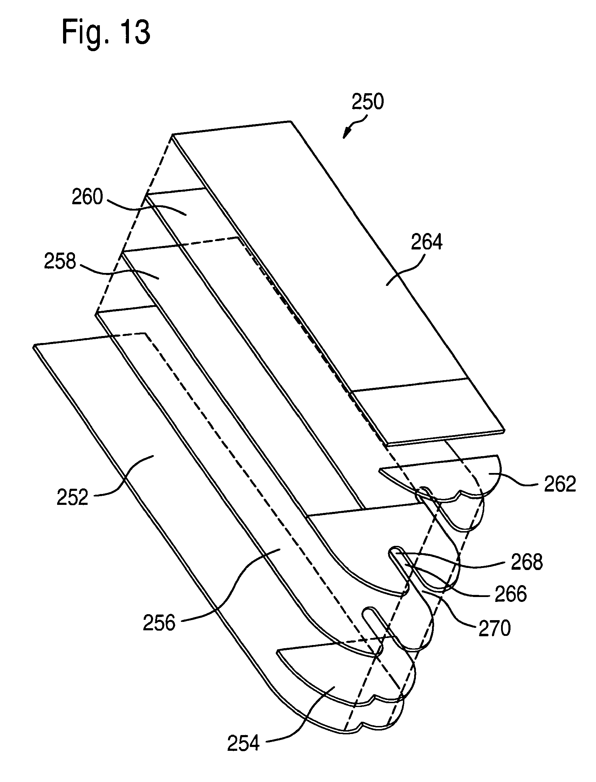

Figure 13 shows an exploded representation of a test section of a test field support designed in the form of a card.

The exploded representation according to Figure 13 shows that a test section 250 has a multilayer structure. A reagent coating 254 is applied on a support sheet 252, although it extends only inside the head region of the test section 250. Above the reagent coating 254, there is a first adhesive layer 256 on which a spacer sheet 258 is in turn applied. A second adhesive layer 260 is applied above the spacer sheet 258, and a hydrophilic layer 262 is likewise applied on it in the head region of the test section 250. A cover layer 264 is applied on top.

A capillary channel 266, the opening of which is denoted by the reference numeral 270, is formed in the first adhesive layer 256 and the spacer sheet 258 lying above it. For example, a bodily fluid such as whole blood or thinned blood can enter the capillary channel 266 at the capillary opening 270 and travel into an electrochemical measuring cell 268 owing to the capillary forces acting there. The electrochemical measuring cell 268, which is formed both in the first adhesive layer 256 and in the spacer sheet 258, is bounded on its upper side by the hydrophilic layer 262 and on its lower side by the reagent coating 254. For economic reasons and in order to save material, the hydrophilic layer 262 and the reagent coating 254 lie only in the head region of the test section 250, which is part of a card-shaped test field support 214 according to the preceding figures.

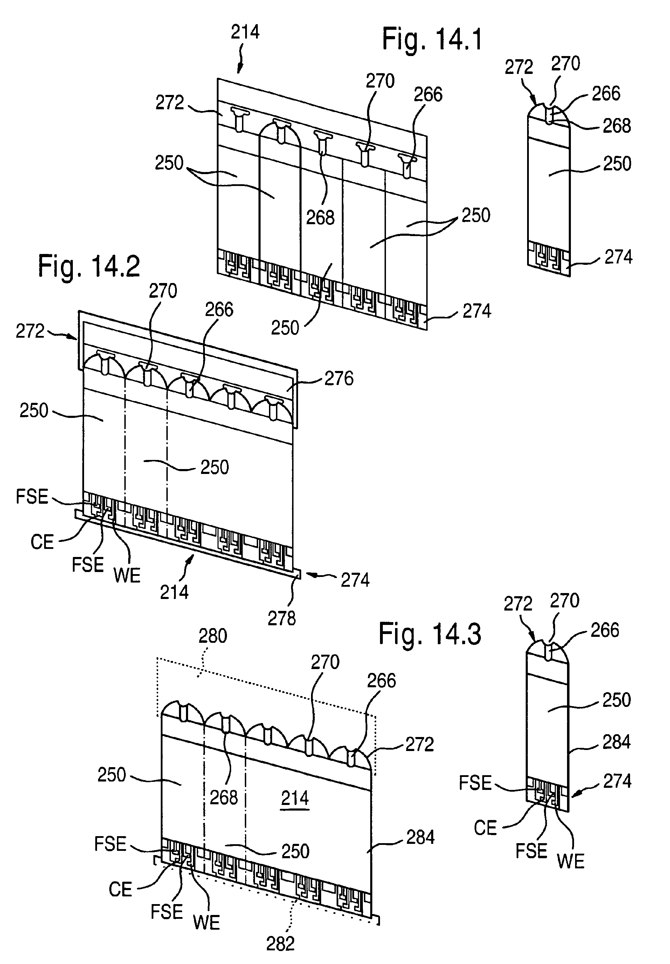

The figure sequence of Figures 14.1, 14.2 and 14.3 shows the structure of a test field support designed in the form of a card.

Figure 14.1 shows that the card-shaped test field support 214 comprises a plurality of test field sections 250 lying next to one another. In the representation according to Figure 14.1, the test field support 214 designed in the form of a card comprises five test sections 250 lying next to one another. Each of the test sections 250 has the capillary openings 270 on the application side 272. In the representation according to Figure 14.1, these are closed by a separable section of the test field support 214 designed in the form of a card. On the contact side 274, there are the electrode terminals for contact with the test field support 214 designed in the form of a card when it is slid into the slot-in slot 220, for example represented in Figure 12, of the slot-in module 202.

Figure 14.1 furthermore shows a test section 250 separated from the test field support 214 designed in the form of a card. In the upper region of the test section 250, the capillary opening 270 is represented as being open, and the capillary channel 266 extends from it to the electrochemical measuring cell 268. The reference numeral 272 denotes the user side, on which the capillary opening 270 is also located; the reference numeral 274 denotes the contact side of the test section 250.

Figure 14.2 likewise shows a test field support designed in the form of a card, although its contact and application sides are protected.

There is a material projection 276 on the application side 272 on the card-shaped test field support 214 according to the representation in Figure 14.2. The same applies to the contact side 274, on which a material projection 278 is likewise formed. The material projection 276 protects the capillary openings 270 on the application side 272, so that no contaminants can enter the capillary channel 266 before use by the user. The material projection 278 is used in order to stabilize the contact side 274 of the test field support 214 designed in the form of a card. The material projections 276 and 278 represented in Figure 14.2 may, for example, be separated simply by bending them before the test field support 214 is inserted into the slot-in module 202. This is necessary, on the one hand, in order to electrically connect the test field support 214 designed in the form of a card to the slot-in module 202 and, on the other hand, in order to permit use of the individual test sections 250 configured lying next to one another.

On the contact side 274 of the test field support 214 designed in the form of a card according to Figure 14.2, there are the - still protected by the material projection 278 - individual electrodes FSE, CE and WE (cf. representation according to Figure 2), by which the individual test sections 250 are electrically connected to the slot-in module 214 and can be evaluated according to the analogue semiconductor switching matrix 54 represented in Figure 3. Here, reference is made to the description of the electrodes FSE, CE and WE in connection with Figure 2.

Figure 14.3 shows a test field support designed in the form of a card, the application side of which and the contact side of which are exposed.

The representation according to Figure 14.3 shows that the material projection 276 provided on the application side 272 in Figure 14.2 is removed, as indicated by the region identified by the reference numeral 280. The same applies to the removal of the material projection 278 on the contact side 274 of the test field support 214 designed in the form of a card. This has a multilayer structure 284, reference being made to the exploded representation according to Figure 13. After separation of the material projection 276 on the application side 276, the capillary openings at which the capillary channel 266 connects with the electrochemical measuring cell 268 are open and can be activated by wetting with a bodily fluid, for example whole blood or thinned blood.

Figure 14.3 furthermore shows an individual test section 250, in which the capillary opening 270 as well as the electrodes FSE, CE and WE are likewise exposed by separating the material projections 276 and 274 (cf. representation according to Fig. 14.2).

### List of Reference Numerals

10 measuring device
12 lower shell
14 upper shell
16 articulated connection
18 folding case
20 opening boundary
22 1^{st} display
24 2^{nd} display
26 flat test field support
28 test field support surface
30 test field array
32 base sheet
34 support sheet
3 6 conductor tracks
38 electrodes

CE counter electrode
WE wettable electrode
FSE 1^{st} filling level electrode
FSE 2^{nd} filling level electrode

40 reagent layer
42 spacer sheet
44 hole
46 measuring chamber/measuring capillary space
48 cover sheet
50 hydrophilic layer
52 vent
54 analogue semiconductor switching matrix
56 CE terminal
58 WE terminal
60 terminal of 1^{st} FSE
62 terminal of 2^{nd} FSE
64 1^{st} switch
66 2^{nd} switch
68 individual test field
70 electrical contact strip
72 shell-side electrical contact strip
74 closed individual test field
76 ring-pull closure
78 cover
80 reception well
82 sealing edge

100 capillary sensor support
102 1^{st} long side
104 capillary sensor
106 2^{nd} long side
108 capillary with opening
110 1^{st} contact region of capillary sensor support
112 2^{nd} contact region of capillary sensor support
140 measuring and analysis device
142 lower part
144 upper part
146 hinge
148 resilient contacts arranged in a row
150 display
152 control buttons
154 cover sleeve
156 retracted state
158 slid-on state

200 slot-in arrangement
202 slot-in module
204 slot-in box
206 gripping piece
208 latching/unlatching
210 gripping side
212 recess
214 test field support
216 light-emitting diodes
218 capillary opening
220 slot-in slot
222 guide surface
224 bounding wall
226 control field
248 keypad
230 display
234 edge of test field support
236 contact region of test field support 214
238 latching element
240 free space
242 slot-in position of test field support 214

250 test section
252 support sheet
254 reagent coating
256 1^{st} adhesive layer
258 spacer sheet
260 2^{nd} adhesive layer
262 hydrophilic layer
264 cover layer
266 capillary channel
268 measuring chamber
270 capillary opening
272 application side
274 contact side
276 material projection on application side
278 material projection on contact side
280 extent of detached material projection 276
282 extent of detached material projection 278
284 layer structure of test section

## Claims

1. Measuring device for analysing a sample liquid with respect to at least one analyte contained in it, having at least one test field support (26, 100, 214) which is housed in it and has a number of individual test fields (68, 104, 250), which are in communication with electrochemical measuring cells (46, 268) in the at least one test field support (26, 100, 214), and having reagents assigned to the electrochemical measuring cells (46, 268), the reaction of which with the sample liquid leads to a measurable change of at least one quantity characteristic of the determination of the at least one analyte, the measuring device (10, 140, 200) comprising evaluation electronics (54), **characterized in that** a plurality of individual test fields (68, 104, 250) on the test field support (26, 100, 214) are accessible to the user after the measuring device (10, 140, 200) has been opened, wherein the measuring device is a portable measuring device and contains a long-term energy store, which supplies energy to evaluation electronics accommodated in the portable measuring device, wherein the measuring device has an analogue semiconductor switching matrix (54) and a microprocessor (µP), via which the individual test field (68, 104, 250) respectively selected by the user can be connected to evaluation electronics.

2. Measuring device according to Claim 1, **characterized in that** the at least one test field support (26, 100, 214) is housed in the measuring device (10, 140, 200) in such way that it can be replaced.

3. Measuring device according to Claim 1, **characterized in that** the position of the individual test fields (68, 104, 250) on the at least one test field support (26, 100, 214) relative to the measuring system is fixed.

4. Measuring device according to Claim 1, **characterized in that** electrodes (38; FSE, CE, WE) of the individual test fields (68, 104, 250) of the at least one test field support (26, 100, 214) are electrically connected simultaneously after the at least one test field support (26, 100, 214) has been inserted in a housing component (12, 142, 202) of the measuring device (10, 140, 200).

5. Measuring device according to Claim 1, **characterized in that** the individual test fields (68) are arranged matricially on a test field support surface (28) of the at least one test field support (26).

6. Measuring device according to Claim 5, **characterized in that** the individual test fields (68) of the at least one test field support (26) are respectively assigned a closure (78), with which a reception well (80) of a respective individual test field (68) is sealed.

7. Measuring device according to Claim 5, **characterized in that** the reception well (80) is in communication with an electrochemical measuring cell (46) in the at least one test field support (26) constructed from a plurality of function layers (32, 34, 40, 42, 48).

8. Measuring device according to Claim 4, **characterized in that** the at least one test field support (26) has an electrical contact (70) which connects with the electrical contact (64) of a lower shell (12) when it is inserted therein.

9. Measuring device according to Claim 1, **characterized in that** it is designed in the form of a case, and has a lower shell (12) and an upper shell (14), on the inside of which displays (22, 24) can be read after the upper shell (14) has been opened.

10. Measuring device according to Claim 5, **characterized in that** the at least one test field support (26) is designed in the form of a card.

11. Measuring device according to Claim 1, **characterized in that** the at least one test field support (100) is designed as a capillary sensor support, which contains a number of neighbouring capillary sensors (104).

12. Measuring device according to Claim 11, **characterized in that** the at least one test field support (100) is a section of a band material constructed from a plurality of function layers.

13. Measuring device according to Claim 11, **characterized in that** it is designed in the form of a bar and comprises a lower part (142) and an upper part (144) articulatedly connected thereto, it being possible to insert at least one test field support (100) which can be electrically connected by resilient contacts (148) arranged on the lower part (142) or on the upper part (144).

14. Measuring device according to Claim 13, **characterized in that** openings of capillaries (108) of the capillary sensors (104) are accessible in the electrically connected state of the at least one test field support (100) with the upper part (144) closed.

15. Measuring device according to Claim 1, **characterized in that** it is designed as a slot-in arrangement (200) and comprises a slot-in box (204) with a latch (208), as well as a slot-in module (202) which can be removed from the slot-in box (204).

16. Measuring device according to Claim 15, **characterized in that** the slot-in module (202) has a recess (212) with slot-in slots (220) laterally bordering the recess (212) in order to accommodate the at least one card-shaped test field support (214).

17. Measuring device according to Claim 16, **characterized in that** the slot-in module (202) has light-emitting diodes (216) in the region of the recess (212), of which one light-emitting diode (216) is respectively assigned to one test field section (250) of the card-shaped test field support (214).

18. Measuring device according to Claim 17, **characterized in that** the at least one card-shaped test field support (214) comprises a plurality of test field sections (250).

19. Measuring device according to Claim 16, **characterized in that** the card-shaped test field support (214) can be electrically connected on a contact side (274) when it has been slid into the slot-in module (202).

20. Measuring device according to Claim 16, **characterized in that** electrode terminals for connection of the electrodes (38; FSE, CE, WE) are provided on the contact side (274) of the card-shaped test field support (214).

21. Measuring device according to Claim 1, **characterized in that** the individual test fields (68, 104, 250) of the card-shaped test field support (26, 214) can be electrically connected simultaneously.

## Patentansprüche

1. Messvorrichtung zur Analyse einer Probenflüssigkeit bezüglich zumindest eines darin enthaltenen Analyten, mit zumindest einer Testfeldstütze (26, 100, 214), die darin untergebracht ist und eine Anzahl von individuellen Testfeldern (68, 104, 250) aufweist, die mit elektrochemischen Messzellen (46, 268) in der zumindest einen Testfeldstütze (26, 100, 214) in Verbindung stehen, und mit Reagenzien, die den elektrochemischen Messzellen (46, 268) zugeordnet sind, deren Reaktion mit der Probenflüssigkeit zu einer messbaren Veränderung zumindest einer Mengeneigenschaft der Bestimmung des zumindest einen Analyten führen, worin die Messvorrichtung (10, 140, 200) eine Auswertungselektronik (54) umfasst, **dadurch gekennzeichnet, dass** eine Vielzahl von individuellen Testfeldern (68, 104, 250) auf der Testfeldstütze (26, 100, 214) für den Anwender zugänglich sind, nachdem die Messvorrichtung (10, 140, 200) geöffnet wurde, worin die Messvorrichtung eine tragbare Messvorrichtung ist und einen Langzeitenergiespeicher aufweist, der die in der tragbaren Messvorrichtung untergebrachte Auswertungselektronik mit Energie versorgt, worin die Messvorrichtung eine Analog-Halbleiter-Schaltmatrix (54) und einem Mikroprozessor (µP) aufweist, über den das jeweils vom Anwender gewählte individuelle Testfeld (68, 104, 250) mit der Auswertungselektronik verbunden werden kann.

2. Messvorrichtung nach Anspruch 1, **dadurch gekennzeichnet, dass** die zumindest eine Testfeldstütze (26, 100, 214) so in der Messvorrichtung (10, 140, 200) untergebracht ist, dass sie ersetzt werden kann.

3. Messvorrichtung nach Anspruch 1, **dadurch gekennzeichnet, dass** die Position der individuellen Testfelder (68, 104, 250) auf der zumindest einen Testfeldstütze (26, 100, 214) bezüglich des Messsystems festgelegt ist.

4. Messvorrichtung nach Anspruch 1, **dadurch gekennzeichnet, dass** Elektroden (38; FSE, CE, WE) der individuellen Testfelder (68, 104, 250) der zumindest einen Testfeldstütze (26, 100, 214) gleichzeitig elektrisch verbunden werden, nachdem die zumindest eine Testfeldstütze (26, 100, 214) in eine Gehäusekomponente (12, 142, 202) der Messvorrichtung (10, 140, 200) eingeführt wurde.

5. Messvorrichtung nach Anspruch 1, **dadurch gekennzeichnet, dass** die individuellen Testfelder (68) matrixförmig auf einer Testfeldstützenoberfläche (28) der zumindest einen Testfeldstütze (26) angeordnet sind.

6. Messvorrichtung nach Anspruch 5, **dadurch gekennzeichnet, dass** die individuellen Testfelder (68) der zumindest einen Testfeldstütze (26) jeweils einem Verschluss (78) zugeordnet sind, mit dem eine Aufnahmemulde (80) eines jeweiligen individuellen Testfeldes (68) verschlossen ist.

7. Messvorrichtung nach Anspruch 5, **dadurch gekennzeichnet, dass** die Aufnahmemulde (80) mit einer elektrochemischen Messzelle (46) in der zumindest einen Testfeldstütze (26) in Verbindung steht, die aus einer Vielzahl von Funktionsschichten (32, 34, 40, 42, 48) konstruiert ist.

8. Messvorrichtung nach Anspruch 4, **dadurch gekennzeichnet, dass** die zumindest eine Testfeldstütze (26) einen elektrischen Kontakt (70) aufweist, der mit dem elektrischen Kontakt (64) einer unteren Halbschale (12) in Verbindung tritt, wenn er darin eingeführt ist.

9. Messvorrichtung nach Anspruch 1, **dadurch gekennzeichnet, dass** sie in Form eines Etuis gestaltet ist und eine untere Halbschale (12) und eine obere Halbschale (14) aufweist, auf deren Innenseite Anzeigen (22, 24) gelesen werden können, nachdem die obere Halbschale (14) geöffnet wurde.

10. Messvorrichtung nach Anspruch 5, **dadurch gekennzeichnet, dass** die zumindest eine Testfeldstütze (26) in Form einer Karte gestaltet ist.

11. Messvorrichtung nach Anspruch 1, **dadurch gekennzeichnet, dass** die zumindest eine Testfeldstütze (100) als Kapillarsensorstütze gestaltet ist, die eine Anzahl von benachbarten Kapillarsensoren (104) enthält.

12. Messvorrichtung nach Anspruch 11, **dadurch gekennzeichnet, dass** die zumindest eine Testfeldstütze (100) ein Bandmaterialabschnitt ist, der aus einer Vielzahl von Funktionsschichten konstruiert ist.

13. Messvorrichtung nach Anspruch 11, **dadurch gekennzeichnet, dass** sie in Form eines Stabs gestaltet ist und ein Unterteil (142) und ein beweglich damit verbundenes Oberteil (144) umfasst, wobei zumindest eine Testfeldstütze (100), die über nachgiebige Kontakte (148), die auf dem Unterteil (142) oder auf dem Oberteil (144) angeordnet sind, elektrisch verbunden werden kann, eingeführt werden kann.

14. Messvorrichtung nach Anspruch 13, **dadurch gekennzeichnet, dass** Öffnungen von Kapillaren (108) der Kapillarsensoren (104) im elektrisch verbundenen Zustand der zumindest einen Testfeldstütze (100) bei geschlossenem Oberteil (144) zugänglich sind.

15. Messvorrichtung nach Anspruch 1, **dadurch gekennzeichnet, dass** sie als Einsteckanordnung (200) gestaltet ist und eine Einsteckbox (204) mit einem Riegel (208) sowie ein Einsteckmodul (202), das aus der Einsteckbox (204) entfernt werden kann, umfasst.

16. Messvorrichtung nach Anspruch 15, **dadurch gekennzeichnet, dass** das Einsteckmodul (202) eine Vertiefung (212) mit Einsteckschlitzen (220) umfasst, die zur Unterbringung der zumindest einen kartenförmigen Testfeldstütze (214) seitlich an die Vertiefung (212) angrenzen.

17. Messvorrichtung nach Anspruch 16, **dadurch gekennzeichnet, dass** das Einsteckmodul (202) Leuchtdioden (216) in der Region der Vertiefung (212) aufweist, von denen jeweils eine Leuchtdiode (216) einem Testfeldabschnitt (250) der kartenförmigen Testfeldstütze (214) zugeordnet ist.

18. Messvorrichtung nach Anspruch 17, **dadurch gekennzeichnet, dass** die zumindest eine kartenförmige Testfeldstütze (214) eine Vielzahl von Testfeldabschnitten (250) umfasst.

19. Messvorrichtung nach Anspruch 16, **dadurch gekennzeichnet, dass** die kartenförmige Testfeldstütze (214) elektrisch mit einer Kontaktseite (274) verbunden werden kann, wenn sie in das Einsteckmodul (202) eingeschoben wurde.

20. Messvorrichtung nach Anspruch 16, **dadurch gekennzeichnet, dass** Elektrodenklemmen zur Verbindung der Elektroden (38; FSE, CE, WE) auf der Kontaktseite (274) der kartenförmigen Testfeldstütze (214) vorgesehen sind.

21. Messvorrichtung nach Anspruch 1, **dadurch gekennzeichnet, dass** die individuellen Testfelder (68, 104, 250) der kartenförmigen Testfeldstütze (26, 214) gleichzeitig elektrisch verbunden werden können.

## Revendications

1. Dispositif de mesure destiné à analyser au moins un analyte que contient un liquide d'échantillon et dans lequel est logé au moins un support (26, 100, 214) de champ de test doté de plusieurs champs de test distincts (68, 104, 250) qui communiquent avec des cellules électrochimiques de mesure (46, 268) prévues dans le ou les supports (26, 100, 214) de champ de test et présentant des réactifs associés aux cellules électrochimiques de mesure (46, 268) dont la réaction avec le liquide d'échantillon entraîne une modification mesurable d'au moins une quantité caractéristique de la présence du ou des analytes, le dispositif de mesure (10, 140, 200) comprenant une électronique d'évaluation (54),
**caractérisé en ce que**
plusieurs champs de test distincts (68, 104, 250) prévus sur le support (26, 100, 214) de champs de test sont accessibles à l'utilisateur après que le dispositif de mesure (10, 140, 200) a été ouvert, le dispositif de mesure étant un dispositif de mesure portable et contenant une réserve d'énergie de longue durée qui délivre de l'énergie à l'électronique d'évaluation logée dans le dispositif portable de mesure, le dispositif de mesure présentant une matrice analogique semi-conductrice de commutation (54) et un microprocesseur (µP) par lequel les différents champs de test distincts (68, 104, 250) sélectionnés par l'utilisateur peuvent être raccordés à l'électronique d'évaluation.

2. Dispositif de mesure selon la revendication 1, **caractérisé en ce que** le ou les supports (26, 100, 214) de champs de test sont logés dans le dispositif de mesure (10, 140, 200) de manière à pouvoir être remplacés.

3. Dispositif de mesure selon la revendication 1, **caractérisé en ce que** la position des différents champs de test (68, 104, 250) situés sur le ou les supports (26, 100, 214) de champs de test par rapport au système de mesure est fixe.

4. Dispositif de mesure selon la revendication 1, **caractérisé en ce que** des électrodes (38; FSE, CE, WE) des différents champs de test (68, 104, 250) prévus sur le ou les supports (26, 100, 214) de champs de test sont raccordées électriquement de manière simultanée après que le ou les supports (26, 100, 214) de champs de test ont été insérés dans un composant de reprise (12, 142, 202) du dispositif de mesure (10, 140, 200).

5. Dispositif de mesure selon la revendication 1, **caractérisé en ce que** les différents champs de test (68) sont agencés en matrice sur une surface (28) de support de champs de test du ou des supports (26) de champs de test.

6. Dispositif de mesure selon la revendication 5, **caractérisé en ce que** les différents champs de test (68) du ou des supports (26) de champs de test sont associés respectivement à une fermeture (78) par laquelle un puits de reprise (80) du champ de test particulier (68) concerné est fermé hermétiquement.

7. Dispositif de mesure selon la revendication 5, **caractérisé en ce que** le puits de reprise (80) communique avec une celle électrochimique de mesure (46) du ou des supports (26) de champs de test constitués de plusieurs couches fonctionnelles (32, 34, 40, 42, 48).

8. Dispositif de mesure selon la revendication 4, **caractérisé en ce que** le ou les supports (26) de champs de test présentent un contact électrique (70) raccordé au contact électrique (64) d'une coque inférieure (12) lorsqu'il y est inséré.

9. Dispositif de mesure selon la revendication 1, **caractérisé en ce qu'**il est conçu sous la forme d'une valise, présente une coquille inférieure (12) et une coquille supérieure (14) à l'intérieur de laquelle des affichages (22, 24) peuvent être lus après que la coquille supérieure (14) a été ouverte.

10. Dispositif de mesure selon la revendication 5, **caractérisé en ce que** le ou les supports (26) de champs de test sont conçus sous la forme d'une carte.

11. Dispositif de mesure selon la revendication 1, **caractérisé en ce que** le ou les supports (100) de champs de test sont conçus sous la forme d'un support de détecteurs capillaires qui contient plusieurs détecteurs capillaires (104) voisins.

12. Dispositif de mesure selon la revendication 11, **caractérisé en ce que** le ou les supports (100) de champs de test sont formés d'une section d'un matériau en ruban constitué de plusieurs couches fonctionnelles.

13. Dispositif de mesure selon la revendication 11, **caractérisé en ce qu'**il est conçu sous la forme d'un barreau et comprend une partie inférieure (142) à laquelle une partie supérieure (144) est reliée de manière articulée, au moins un support (100) de champs de test qui peut être raccordé électriquement par des contacts élastiques (148) agencés sur la partie inférieure (142) ou sur la partie supérieure (144) insérable.

14. Dispositif de mesure selon la revendication 13, **caractérisé en ce que** les ouvertures des capillaires (108) des détecteurs capillaires (104) sont accessibles lorsque le ou les supports (100) de champs de test sont raccordés électriquement et que la partie supérieure (144) est fermée.

15. Dispositif de mesure selon la revendication 1, **caractérisé en ce qu'**il est conçu comme agencement (200) insérable dans une fente et qu'il comprend un boîtier insérable (204) doté d'un verrou (208) ainsi que d'un module insérable (202) qui peut être retiré du boîtier d'insertion (204).

16. Dispositif de mesure selon la revendication 15, **caractérisé en ce que** le module d'insertion (202) présente un creux (212) doté de fentes d'insertion (220) qui bordent latéralement le creux (212) de manière à loger le ou les supports (214) de champs de test en forme de carte.

17. Dispositif de mesure selon la revendication 16, **caractérisé en ce que** le module d'insertion (202) présente des diodes luminescentes (216) au niveau du creux (212), chaque diode luminescente (216) étant associée à une section (250) de champs de test du support (214) de champs de test en forme de carte.

18. Dispositif de mesure selon la revendication 17, **caractérisé en ce que** le ou les supports (214) de champs de test en forme de carte comprennent plusieurs sections (250) de champ de test.

19. Dispositif de mesure selon la revendication 16, **caractérisé en ce que** le support (214) de champs de test en forme de carte peut être raccordé électriquement à un côté de contact (274) lorsqu'il a été inséré dans le module d'insertion (202).

20. Dispositif de mesure selon la revendication 16, **caractérisé en ce que** des bornes d'électrodes destinées à être raccordées aux électrodes (38; FSE, CE, WE) sont prévues sur le côté de contact (274) du support (214) de champs de test en forme de carte.

21. Dispositif de mesure selon la revendication 1, **caractérisé en ce que** les différents champs de test (68, 104, 250) du support (26, 214) de champs de test en forme de carte peuvent être raccordés électriquement de manière simultanée.
